(19) Europäisches Patentamt
European Patent Office
Office européen des brevets

(11) EP 3 510 991 A1

# (12) EUROPEAN PATENT APPLICATION

published in accordance with Art. 153(4) EPC

(43) Date of publication:
**17.07.2019 Bulletin 2019/29**

(21) Application number: **17849080.1**

(22) Date of filing: **06.09.2017**

(51) Int Cl.:
***A61K 8/368*** *(2006.01)* ***A61K 8/60*** *(2006.01)*
***A23L 33/10*** *(2016.01)* ***A61K 31/192*** *(2006.01)*
***A61K 31/704*** *(2006.01)* ***A61Q 19/00*** *(2006.01)*
***A61Q 19/08*** *(2006.01)*

(86) International application number:
**PCT/KR2017/009767**

(87) International publication number:
**WO 2018/048196 (15.03.2018 Gazette 2018/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **08.09.2016 KR 20160115548**

(71) Applicant: **Amorepacific Corporation**
**Seoul 04386 (KR)**

(72) Inventors:
- **KANG, Young-Gyu**
  **Yongin-si**
  **Gyeonggi-do 17074 (KR)**
- **PARK, Nok Hyun**
  **Yongin-si**
  **Gyeonggi-do 17074 (KR)**
- **PARK, Jun Seong**
  **Yongin-si**
  **Gyeonggi-do 17074 (KR)**

(74) Representative: **Agasse, Stéphane et al**
**Cabinet GERMAIN & MAUREAU**
**B.P. 6153**
**69466 Lyon Cedex 06 (FR)**

## (54) SKIN ANTI-AGING COMPOSITION CONTAINING DEHYDROGENATED ABIETIC ACID AND COMPOUND K

(57) The present specification provides a composition for preventing skin aging, containing, as active ingredients: dehydrogenated abietic acid, a stereoisomer thereof, a salt thereof, a hydrate thereof, or a solvate thereof; and compound K represented by chemical formula (2), a stereoisomer thereof, a salt thereof, a hydrate thereof, or a solvate thereof, thereby having excellent skin cell proliferation effects, collagen production promotion effects, and Lipofuscin accumulation inhibition effects, while not causing any skin side effects.

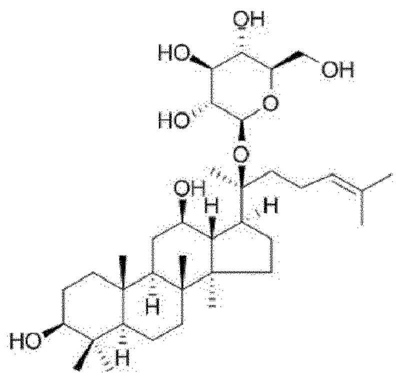

[Formula 2]

EP 3 510 991 A1

## Description

[FIELD OF THE INVENTION]

The present application relates to a composition for anti-aging of a skin comprising dehydroabietic acid and compound K as active ingredients.

[BACKGROUND OF THE INVENTION]

The skin is a primary defense barrier of the human body, which functions to protect various organs in the body from changes in temperature and humidity, and external environmental stimuli such as UV and pollutants, and plays an important role in maintaining homeostasis in vivo. However, excessive physical and chemical stimuli from the external environment, stresses, nutritional deficiency and the like deteriorate the normal function of the skin and accelerate skin aging phenomena such as firmness loss, keratinization, wrinkle formation and the like. To prevent such phenomena and to keep the skin healthier and more elastic, there have been efforts to use cosmetics containing physiologically active substances obtained from various plants, microorganisms and the like, so as to maintain the intrinsic function of the skin and activate skin cells, thus effectively inhibiting skin aging.

However, existing cosmetic raw materials have various problems in that they mostly have insufficient effects or cause side effects on the skin.

Citation List

Patent Literature

Patent Literature 1: Korean Patent No. 10-1349248
Patent Literature 2: Korean Patent Laid-Open No. 10-2014-0003198
Patent Literature 3: Korean Patent Laid-Open No. 10-2006-0104161
Patent Literature 4: Korean Patent No. 10-0882780
Patent Literature 5: Korean Patent No. 10-1401658
Patent Literature 6: Korean Patent No. 10-1140039

[TECHNICAL PROBLEM]

In order to solve the problems, the present invention provides a composition for preventing skin aging, which contains, as active ingredients, dehydroabietic acid and compound K, and thus which has an excellent effect of promoting skin cell proliferation and collagen production and inhibiting lipofuscin accumulation without causing side effects on the skin.

In one aspect, an objective of the present invention is to provide a composition for anti-aging of the skin comprising, as active ingredients, dehydroabietic acid, a stereoisomer thereof, a salt thereof, a hydrate thereof or a solvate thereof; and compound K, a stereoisomer thereof, a salt thereof, a hydrate thereof or a solvate thereof.

In one aspect, an objective of the present invention is to provide a composition for reducing skin wrinkles comprising, as active ingredients, dehydroabietic acid, a stereoisomer thereof, a salt thereof, a hydrate thereof or a solvate thereof; and compound K, a stereoisomer thereof, a salt thereof, a hydrate thereof or a solvate thereof.

In one aspect, an objective of the present invention is to provide a composition for enhancing skin elasticity comprising, as active ingredients, dehydroabietic acid, a stereoisomer thereof, a salt thereof, a hydrate thereof or a solvate thereof; and compound K, a stereoisomer thereof, a salt thereof, a hydrate thereof or a solvate thereof.

[TECHNICAL SOLUTION]

In one aspect, the present invention provides a composition for anti-aging of the skin comprising, as active ingredients, dehydroabietic acid, a stereoisomer thereof, a salt thereof, a hydrate thereof or a solvate thereof; and compound K, a stereoisomer thereof, a salt thereof, a hydrate thereof or a solvate thereof.

In one aspect of the present invention, a weight ratio of dehydroabietic acid, a stereoisomer thereof, a salt thereof, a hydrate thereof or a solvate thereof; and compound K, a stereoisomer thereof, a salt thereof, a hydrate thereof or a solvate thereof in the composition is 1: 1 to 15.

In one aspect of the present invention, the weight ratio is 1: 5 to 10.

In one aspect of the present invention, a content of the active ingredients in the composition is 0.0001 to 10 wt% based on the total weight of the composition.
In one aspect of the present invention, the composition is a composition for reducing skin wrinkles.
In one aspect of the present invention, the composition is a composition for enhancing skin elasticity.
In one aspect of the present invention, the composition is a composition for anti-aging of the skin that prevents skin aging by proliferating fibroblasts.
In one aspect of the present invention, the composition is a composition for anti-aging of the skin that prevents skin aging by promoting collagen biosynthesis.
In one aspect of the present invention, the composition is for a composition for anti-aging of the skin that prevents skin aging by inhibiting lipofuscin accumulation in the skin.
In one aspect of the present invention, the composition is a cosmetic composition.
In one aspect of the present invention, the composition is a food composition.
In one aspect of the present invention, the composition is a pharmaceutical composition.

[EFFECTS OF THE INVENTION]

The composition according to one aspect of the present invention exhibits an excellent effect of reducing skin aging.
The composition according to one aspect of the present invention exhibits an excellent effect of inhibiting skin aging.
The composition according to one aspect of the present invention exhibits an excellent anti-aging effect on the skin without causing side effects on the skin.
The composition according to one aspect of the present invention exhibits an excellent effect of proliferating skin cells without causing side effects on the skin.
The composition according to one aspect of the present invention exhibits an excellent effect of reducing skin wrinkles without causing side effects on the skin.
The composition according to one aspect of the present invention exhibits an excellent effect of enhancing skin elasticity without causing side effects on the skin.
The composition according to one aspect of the present invention exhibits an excellent effect of promoting collagen biosynthesis without causing side effects on the skin.
The composition according to one aspect of the present invention exhibits an excellent effect of inhibiting lipofuscin accumulation without causing side effects on the skin.
The composition according to one aspect of the present invention exhibits a superior anti-aging effect on the skin compared to each of dehydroabietic acid and compound K.

[DETAILED DESCRIPTION OF THE PREFERRED EMBODIMENTS]

Hereinafter, the present invention will be described in further detail.
One aspect of the present invention is to provide a composition for preventing skin aging, which comprises, as active ingredients, dehydroabietic acid and compound K, and thus which has an excellent effect of promoting skin cell proliferation and collagen production and inhibiting lipofuscin accumulation without causing side effects on the skin. In order to achieve the above objective, one aspect of the present invention provides a composition for anti-aging of the skin comprising, as active ingredients, dehydroabietic acid represented by Formula 1 below and compound K represented by Formula 2 below.
Dehydroabietic acid as described herein may be represented by the following Formula 1:

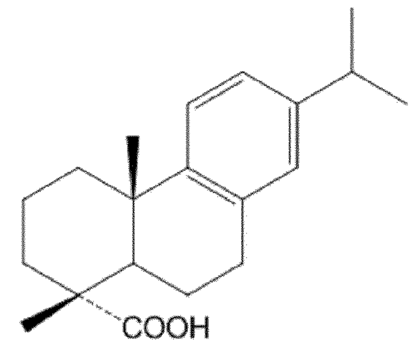

[Formula 1]

Compound K as described herein may be represented by the following Formula 2:

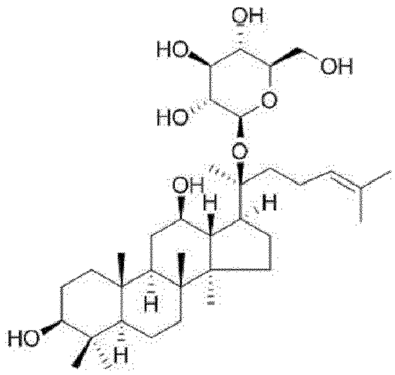

[Formula 2]

One aspect of the present invention provides a composition for anti-aging of the skin comprising, as active ingredients, dehydroabietic acid, a stereoisomer thereof, a salt thereof, a hydrate thereof or a solvate thereof; and compound K, a stereoisomer thereof, a salt thereof, a hydrate thereof or a solvate thereof.

As used herein, the term "stereoisomer" refers to isomers that have the same molecular formula and sequence of bonded atoms, but differ in the three-dimensional orientations of their atoms in space. It may be a diastereomer or an optical isomer. In addition, the diastereomer may be a geometrical isomer or a diastereomer that is not a geometrical isomer.

As used herein, the term "salt" refers to a "pharmaceutically or cosmetically acceptable salt". The term "pharmaceutically or cosmetically acceptable" means that which is useful in preparing a pharmaceutical composition that is generally safe, nontoxic and biologically or otherwise desirable and includes that which is acceptable for veterinary use as well as human pharmaceutical and cosmetic uses. As used herein, the term "pharmaceutically or cosmetically acceptable salts" means salts which are pharmaceutically or cosmetically acceptable, as defined above, and which possess the desired pharmacological activity. Such salts include (1) acid addition salts formed with inorganic acids such as hydrochloric acid, hydrobromic acid, sulfuric acid, nitric acid, phosphoric acid, and the like; or with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2,-ethanedisulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]oct-2-ene-l-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts that are formed when an acidic proton present in a parent compound is replaced.

As used herein, the term "hydrate" means a "pharmaceutically or cosmetically acceptable hydrate". The "hydrate" exists when the compound of the present invention contains water. The hydrate may contain one or more water molecules per molecule of the compound of the present invention. Illustrative non-limiting examples include monohydrates, dehydrates, trihydrates and tetrahydrates. The hydrate may contain at least one molecule of compound of the

present invention per molecule of water. Illustrative non-limiting examples include semi-hydrates. In an embodiment, the water may be held in the crystal in various ways, and thus the water molecule may occupy lattice positions in the crystal, or may form a bond with a salt of the compound as described herein. The hydrate must be "acceptable" in the sense of not being deleterious to the recipient thereof.

As used herein, the term "solvate" means a "pharmaceutically or cosmetically acceptable solvate". The term "solvate" means that the compound of the invention contains one or more pharmaceutically acceptable solvents. The solvate may contain one or more molecules of solvent per molecule of compound of the present invention, or may contain one or more molecules of compound of the invention per molecule of solvent. In one embodiment, the solvent may be held in the crystal in various ways, and thus the solvent molecule may occupy lattice positions in the crystal, or may form a bond with a salt of the compound as described herein.

As used herein, "anti-aging of the skin" refers to the ability to slow down, stop or prevent the aging process known in the art. Specifically, it may mean the ability to enhance skin elasticity and reduce wrinkles by proliferating skin cells and skin collagen biosynthesis and inhibiting lipofuscin accumulation, although not limited thereto.

In one aspect of the present invention, the weight ratio of dehydroabietic acid, a stereoisomer thereof, a salt thereof, a hydrate thereof or a solvate thereof; and compound K, a stereoisomer thereof, a salt thereof, a hydrate thereof or a solvate thereof in the composition may be 1: 1 to 15. Specifically, the weight ratio may be 1: 1 or more, 1: 2 or more, 1: 3 or more, 1: 4 or more, 1: 5 or more, 1: 5.5 or more, or 1: 6 or more. In addition, the weight ratio may be 1: 15 or less, 1: 14 or less, 1: 13 or less, 1: 12 or less, 1: 11 or less, 1:10 or less, 1: 9 or less, 1: 8 or less, or 1: 7.5 or less. Preferably, the weight ratio of dehydroabietic acid and compound K in the composition may be 1: 5 to 10. When the weight ratio of dehydroabietic acid and compound K is within the above range, the effects of proliferating skin cells, promoting collagen biosynthesis and inhibiting lipofuscin accumulation in skin cells are remarkably excellent. In addition, when the weight ratio of dehydroabietic acid: compound K is 1: 1 or more, lipofuscin accumulation is further inhibited as compared with the case where the weight ratio is 1: less than 1. In addition, when the weight ratio of dehydroabietic acid: compound K is 1:15 or less, lipofuscin accumulation is further increased as compared with the case where the weight ratio is 1: more than 15.

In one aspect of the present invention, the content of the active ingredients in the composition may be 0.0001 to 10 wt% based on the total weight of the composition. Specifically, the content of the active ingredients may be 0.0001 wt% or more, 0.0005 wt% or more, 0.001 wt% or more, 0.005 wt% or more, 0.01 wt% or more, 0.05 wt% or more, 0.1 wt% or more, 0.5 wt% or more, 1 wt% or more, 2 wt% or more, or 5 wt% or more based on the total weight of the composition. In addition, the content of the active ingredients may be 10 wt% or less, 9 wt% or less, 8 wt% or less, 7 wt% or less, 6 wt% or less, 5 wt% or less, 3 wt% or less, 1 wt% or less, 0.1 wt% or less, 0.01 wt% or less, or 0.001 wt% or less based on the total weight of the composition. If the content of the active ingredients is less than 0.0001 wt%, the effects of anti-aging of skin, skin wrinkle reduction and skin elasticity enhancement will be insignificant. If the content of the active ingredients is more than 10 wt%, an increase in the content will not lead to a significant increase in the effects, and thus will not be cost-effective.

In one aspect of the present invention, the composition may be a composition for reducing skin wrinkles.

In one aspect of the present invention, the composition may be a composition for enhancing skin elasticity.

In one aspect of the present invention, the composition may be a composition for anti-aging of the skin that prevents skin aging by proliferating fibroblasts.

In one aspect of the present invention, the composition may be a composition for anti-aging of the skin that prevents skin aging by promoting collagen biosynthesis.

In one aspect of the present invention, the composition may be a composition for anti-aging of the skin that prevents skin aging by inhibiting lipofuscin accumulation in the skin. Lipofuscin is known to interfere with DNA and RNA synthesis and protein synthesis and destroy cellular enzymes required for important chemical metabolic processes. Thus, the inhibition of lipofuscin is an important factor in anti-aging of the skin.

In one aspect of the present invention, the composition may be a cosmetic composition. The formulation of the cosmetic composition is not specifically limited, and can be suitably selected depending on the intended use. For example, it may be one or more formulations selected from the group consisting of skin lotion, skin softer, skin toner, astringent, lotion, milk lotion, moisture lotion, nourishing lotion, massage cream, nourishing cream, moisture cream, hand cream, foundation, essence, nourishing essence, pack, soap, cleansing foam, cleansing lotion, cleansing cream, body lotion, and body cleanser, although not limited thereto.

When the formulation of the present invention is a paste, a cream or a gel, it may contain, as a carrier ingredient, animal fiber, plant fiber, wax, paraffin, starch, tragacanth, cellulose derivatives, polyethylene glycol, silicone, bentonite, silica, talc, or zinc oxide, etc.

When the formulation of the present invention is a powder or a spray, it may contain, as a carrier ingredient, lactose, talc, silica, aluminum hydroxide, calcium silicate or polyamide powder. Particularly, when the formulation is a spray, it may further contain a propellant such as chlorofluorohydrocarbon, propane/butane or dimethyl ether.

When the formulation of the present invention is a solution or an emulsion, it may contain, as a carrier ingredient,

a solvent, a solubilizer or an emulsifier. Examples of this carrier ingredient include water, ethanol, isopropanol, ethyl carbonate, ethyl acetate, benzyl alcohol, benzyl benzoate, propylene glycol, 1,3-butyl glycol oil, glycerol aliphatic ester, polyethylene glycol, or fatty acid ester of sorbitan.

When the formulation of the present invention is a suspension, it may contain, as a carrier ingredient, a liquid diluent such as water, ethanol or propylene glycol, a suspending agent such as ethoxylated isostearyl alcohol, polyoxyethylene sorbitol ester or polyoxyethylene sorbitan ester, microcrystalline cellulose, aluminum metahydroxide, bentonite, agar, or tragacanth, etc.

When the formulation of the present invention is a surfactant-containing cleanser, it may contain, as a carrier ingredient, aliphatic alcohol sulfate, aliphatic alcohol ether sulfate, sulfosuccinic monoester, isethionate, imidazolinium derivatives, methyl taurate, sarcosinate, fatty acid amide ether sulfate, alkyl amidobetaine, aliphatic alcohol, fatty acid glyceride, fatty acid diethanolamide, vegetable oil, lanolin derivatives, or ethoxylated glycerol fatty acid ester.

The content of the active ingredient is not specifically limited, but it may be 0.0001 to 10 wt% based on the total weight of the composition. When the content of the active ingredients is within the above range, the composition can exhibit excellent effects without side effects.

In one aspect of the present invention, the cosmetic composition may further contain, in addition to the active ingredients, a functional additive and other ingredients that are generally used in cosmetic compositions. The functional additive may be selected from the group consisting of water-soluble vitamins, oil-soluble vitamins, polypeptides, polysaccharides, sphingolipids, and seaweed extracts.

In one aspect of the present invention, the cosmetic composition of the present invention may, if necessary, contain, in addition to the functional additive, ingredients that are generally used in cosmetic compositions. Examples of such additional ingredients include oil and fat ingredients, moisturizers, emollients, surfactants, organic and inorganic pigments, organic powder, UV absorbers, preservatives, sterilizers, antioxidants, plant extracts, pH adjusters, alcohols, colorants, fragrance, blood circulation stimulants, skin coolers, deodorants, purified water, etc.

In one aspect of the present invention, the composition may be a composition for external application to the skin.

In one aspect of the present invention, the composition may be a pharmaceutical composition. The pharmaceutical composition according to one aspect of the present invention may be formulated into various oral or parenteral dosage formulations. When the composition is formulated, generally used diluents or excipients such as fillers, extenders, binders, wetting agents, disintegrants, surfactants, etc. are used. Solid preparations for oral administration include tablets, pills, powders, granules, soft or hard capsules, etc., and are prepared by mixing at least one compound with at least one excipient, for example, starch, calcium carbonate, sucrose or lactose, gelatin, etc. Further, lubricants such as magnesium stearate, talc, etc. are used in addition to simple excipients. Liquid preparations for oral administration include suspensions, solutions, emulsions, syrups, etc., and various excipients such as a wetting agent, a sweetener, a flavoring agent, a preservative, etc. as well as water and liquid paraffin, which are generally used simple diluents, may be added. Formulations for parenteral administration include sterile aqueous solutions, non-aqueous solvents, suspensions, emulsions, freeze-dried formulations, and suppositories. Examples of the non-aqueous solvents or the suspensions include propylene glycol, polyethylene glycol, vegetable oil such as olive oil, injectable esters such as ethyloleate, etc. Examples of the matrix for suppositories include witepsol, macrogol, tween 61, cacao butter, laurinum, glycerogelatin, etc.

In one aspect of the present invention, the active ingredients of the pharmaceutical dosage form of the composition of the present invention may be in the form of their pharmaceutically acceptable salts. In addition, the active ingredients of the composition may be administered alone or in combination with other pharmaceutically active compounds or may be used in the form of an appropriate combination. The salt is not specifically limited as long as it is pharmaceutically acceptable. For example, hydrochloric acid, sulfuric acid, nitric acid, phosphoric acid, hydrofluoric acid, hydrobromic acid, formic acid, acetic acid, tartaric acid, lactic acid, citric acid, fumaric acid, maleic acid, succinic acid, methanesulfonic acid, benzenesulfonic acid, toluenesulfonic acid, naphthalenesulfonic acid, etc. may be used.

In one aspect of the present invention, determination on dosage of the active ingredients is within the knowledge of those skilled in the art. The daily dose of the active ingredients may vary depending on various factors, including the severity of the symptom, the time of onset of the symptom, the subject's age and health conditions, complications, etc. For an adult, the composition may generally be administered at a dose of 1 $\mu$g/kg to 200 mg/kg, preferably 50 $\mu$g/kg to 50 mg/kg, 1-3 times a day. The dose does not limit the scope of the present invention in any way.

The pharmaceutical composition according to one aspect of the present invention may be formulated into any pharmaceutically appropriate form including oral formulations such as powder, granule, tablet, soft or hard capsule, suspension, emulsion, syrup, aerosol, etc., agents for external application to the skin such as ointment, cream, etc., suppository, injection, sterile solution for injection, etc. according to commonly employed methods. Preferably, it may be formulated into an injection or an agent for external application to the skin.

The composition according to one aspect of the present invention may be administered to mammals including rat, mouse, cattle, human, etc. via various routes including parenteral and oral routes. All types of administration may be expected. For example, it may be administered orally, transdermally, rectally, intravenously, intramuscularly, subcutaneously, intrauterinally, or intracerebroventricularly.

The composition according to one aspect the present invention may be administered via various routes that can be easily selected by those skilled in the art. In particular, the pharmaceutical composition according to one aspect of the present invention may be applied on the skin as an agent for external application to the skin.

In one aspect of the present invention, the composition may be a food composition. In one aspect of the present invention, the food composition includes a health food composition. The composition according to one aspect of the present invention may be provided as various types of food additives or functional foods. For example, it may be formulated into fermented milk, cheese, yogurt, juice, probiotics, tablets, granules, drinks, caramels, diet bars, etc. It may be processed into general tea leaves, tea bags, health supplements and the like, or in the form of various other food additives. In one embodiment, the composition may contain other ingredients that can provide synergic effect without negatively affecting the desired main effect, etc. For example, in order to improve the properties, it may further comprise an additive such as flavoring, colorant, sterilizer, antioxidant, preservative, moisturizer, thickener, mineral salt, emulsifier, synthetic polymer, etc. In addition, it may further comprise an auxiliary ingredient such as water-soluble vitamin, oil-soluble vitamin, polypeptide, polysaccharide, seaweed extract, etc. These ingredients may be appropriately selected by those skilled in the art considering the formulation or use. The amount of these ingredients may be determined within a range not negatively affecting the purpose and effect of the present invention. For example, the content of the ingredients may be 0.01- 5 wt%, for example, 0.01-3 wt%, based on the total weight of the composition. The composition according to the present invention may be formulated into various forms such as solutions, emulsions, viscous mixtures, tablets, powders and the like. The composition may be administered according to various methods such as simple drinking, injection, spraying or squeezing.

Hereinafter, the present invention will be described in further detail with reference to examples. It will be apparent to those skilled in the art that these examples are for illustrative purposes only and are not intended to limit the scope of the present invention.

Example 1 and Comparative Examples 1 and 2

The dehydroabietic acid of Formula 1 above purchased from Ramidus AB社 was used in Comparative Example 1, the compound K of Formula 2 above purchased from Ambo Institute 社 was used in Comparative Example 2 and a mixture obtained by mixing the dehydroabietic acid and the compound K at a weight ratio of 1: 7 was used in Example 1. Test Examples 1 to 3 were conducted for the samples of Comparative Examples 1 and 2 and Example 1.

Test Example 1: Effect on Skin Cell Proliferation

In order to examine the effect on skin cell proliferation that reduces wrinkles and enhances skin elasticity, human normal fibroblasts were seeded into a 96-well microplate at a density of $1\times10^4$ cells/well, and incubated in DMEM (Dulbecco's Modified Eagle's Medium) medium for 24 hours. After incubation, the medium was replaced with serum-free DMEM media containing 10 ppm of each of dehydroabietic acid (Comparative Example 1), compound K (Comparative Example 2), and a mixture of dehydroabietic acid and compound K (weight ratio of 1: 7; Example 1), and then the cells were further incubated for 24 hours. Then, 10 μl of 3-(4,5-dimethylthiazol-2-yl)-2,5-diphenyl tetrazolium (MTT, 5 mg/ml) was added to each well, which was then allowed to stand for 4 hours, followed by removal of the medium. Then, 100 μl of dimethyl sulfoxide solution was added to each well, and the absorbance at 570 nm was measured with a microplate reader.

Based on the experiment results, the cell proliferation effect was calculated using the following equation 1. The results of the calculation are shown in Table 1 below.

[Equation 1]

$$\text{Cell proliferation effect (\%)} = \left[\frac{\text{absorbance of group treated with sample} - \text{absorbance of control group}}{\text{absorbance of control group}}\right] \times 100$$

Table 1: Cell proliferation effect

| Ingredient name (concentration) | Absorbance (570nm) | Cell proliferation effect (%) |
|---|---|---|
| Control group (0 ppm) | 0.355 | - |
| Comparative Example 1: dehydroabietic acid (10 ppm) | 0.546 | 53.8 |
| Comparative Example 2: compound K (10 ppm) | 0.520 | 46.5 |
| Example 1: a mixture of dehydroabietic acid and compound K (1: 7) (10 ppm) | 0.644 | 81.4 |

As can be seen from the results in Table 1 above, the mixture of dehydroabietic acid and compound K (Example 1) shows a 1.51 times and 1.75 times higher skin cell proliferation effect than the same concentration of dehydroabietic acid (Comparative Example 1) and compound K (Comparative Example 2), respectively.

Test Example 2: Effect on Promotion of Collagen Biosynthesis

In order to examine the effect on the promotion of collagen biosynthesis that reduces wrinkles and enhances skin elasticity, human normal fibroblasts were seeded into a 96-well microplate at a density of $1\times10^4$ cells/well, and incubated in DMEM (Dulbecco's Modified Eagle's Medium) medium for 24 hours.

After incubation, the medium was replaced with serum-free DMEM media containing 10 ppm of each of dehydroabietic acid (Comparative Example 1), compound K (Comparative Example 2), and a mixture of dehydroabietic acid and compound K (weight ratio of 1: 7; Example 1) and then the cells were further incubated for 48 hours. At 24 hours before completion of the incubation, 10 $\mu$M ascorbic acid as the positive control was added to each well to promote collagen synthesis. After completion, each well was washed, and the medium was replaced with serum-free DMEM medium, after which the cells were further incubated for 24 hours. After incubation, the supernatant of each well was collected, and the amount of procollagen type-I C-peptide (PICP) in the supernatant was measured using a kit (Takara, Kyoto, Japan) to determine the amount of newly synthesized collagen. The amount of PICP that is the amount of collagen synthesized is expressed in the unit of ng/$2\times10^4$ cells in Table 2 below.

Table 2: Effect of promoting collagen biosynthesis

| Ingredient name (concentration) | Amount of collagen synthesized (ng/ $2\times10^4$) |
|---|---|
| Control group (0 ppm) | 125 |
| Ascorbic acid (10 ppm) | 186 |
| Comparative Example 1: dehydroabietic acid (10 ppm) | 174 |
| Comparative Example 2: compound K (10 ppm) | 162 |
| Example 1: a mixture of dehydroabietic acid and compound K (1: 7)(10 ppm) | 289 |

As can be seen from the results in Table 2 above, the mixture of dehydroabietic acid and compound K (Example 1) shows a 1.66 times and 1.78 times higher collagen biosynthesis promotion effect than the same concentration of dehydroabietic acid (Comparative Example 1) and compound K (Comparative Example 2), respectively.

Test Example 3: Effect on Inhibition of Lipofuscin Accumulation in the Skin Cells

In order to examine the effect on the inhibition of lipofuscin accumulation that prevents aging, human normal fibroblasts were seeded into a 96-well microplate at a density of $1\times10^4$ cells/well, and incubated in DMEM (Dulbecco's Modified Eagle's Medium) medium for 24 hours.

After incubation, the medium was replaced with serum-free DMEM media containing 10 ppm of each of dehydroabietic acid (Comparative Example 1), compound K (Comparative Example 2), and a mixture of dehydroabietic acid and compound K (weight ratio of 1: 7; Example 1). Then, the cells were subcultured in a fresh medium every 48 hours. After 10 passages of subculture, the fluorescence of the self-luminescent protein lipofuscin in the cells was measured using a microplate reader with the excitation wavelength of 360 nm and the emission wavelength of 570 nm.

Based on the experiment results, the effect on inhibition of lipofuscin accumulation was calculated using the following equation 2. The results of the calculation are shown in Table 3 below.

[Equation 2]

$$\text{Effect of inhibition of lipofuscin accumulation (\%)} = \left[\frac{\text{absorbance of control group} - \text{absorbance of group treated with sample}}{\text{absorbance of control group}}\right] \times 100$$

Table 3: Effect of inhibition of lipofuscin accumulation

| Ingredient name (concentration) | Absorbance (570nm) | Lipofusin accumulation inhibition rate (%) |
|---|---|---|
| Control group (0 ppm) | 0.650 | - |
| Comparative Example 1: dehydroabietic acid (10 ppm) | 0.465 | 28.5 |
| Comparative Example 2: compound K (10 ppm) | 0.448 | 31.1 |
| Example 1: a mixture of dehydroabietic acid and compound K (1: 7)(10 ppm) | 0.310 | 52.3 |

As can be seen from the results in Table 3 above, the mixture of dehydroabietic acid and compound K (Example 1) shows a 1.83 times and 1.69 times higher inhibitory effect on lipofuscin accumulation than the same concentration of dehydroabietic acid (Comparative Example 1) and compound K (Comparative Example 2), respectively.

Hereinafter, formulation examples of the cosmetic composition having an anti-aging effect on the skin according to one aspect of the present invention will be described. However, it may be formulated into various other forms. These examples are for illustrative purposes only and are not intended to limit the scope of the present invention.

Formulation Example 1: Skin toner

Skin toner is prepared according to a conventional method with the composition shown in Table 4 below.

Table 4

| Ingredient | Content (wt%) |
|---|---|
| Example 1 | 0.1 |
| Glycerin | 3.0 |
| Butylene glycol | 2.0 |
| Propylene glycol | 2.0 |
| Carboxyvinyl polymer | 0.1 |
| PEG 12 nonylphenyl ether | 0.2 |
| Polysorbate 80 | 0.4 |
| Ethanol | 10.0 |
| Triethanolamine | 0.1 |
| Preservative, colorant, fragrance | q.s. |
| Purified water | Balance |

Formulation Example 2: Nourishing cream

Nourishing cream is prepared according to a conventional method with the composition shown in Table 5 below.

Table 5

| Ingredient | Content (wt %) |
|---|---|
| Example 1 | 2.0 |
| Polysorbate 60 | 1.5 |
| Sorbitan sesquioleate | 0.5 |
| PEG 60 hydrogenated castor oil | 2.0 |
| Liquid paraffin | 10.0 |
| Squalane | 5.0 |
| Caprylic/capric triglyceride | 5.0 |
| Glycerin | 5.0 |
| Butylene glycol | 3.0 |
| Propylene glycol | 3.0 |
| Triethanolamine | 0.2 |
| Preservative, colorant, fragrance | q.s. |
| Purified water | Balance |

Formulation Example 3: Massage cream

Massage cream is prepared according to a conventional method with the composition shown in Table 6 below.

Table 6

| Ingredient | Content (wt %) |
|---|---|
| Example 1 | 1.0 |
| Wax | 10.0 |
| Polysorbate 60 | 1.5 |
| PEG 60 hydrogenated castor oil | 2.0 |
| Sorbitan sesquioleate | 0.8 |
| Liquid paraffin | 40.0 |
| Squalane | 5.0 |
| Caprylic/capric triglyceride | 4.0 |
| Glycerin | 5.0 |

Formulation Example 4: Pack

Pack is prepared according to a conventional method with the composition shown in Table 7 below.

Table 7

| Ingredient | Content (wt %) |
|---|---|
| Example 1 | 5.0 |
| Polyvinyl alcohol | 13.00 |

(continued)

| Ingredient | Content (wt %) |
|---|---|
| L-ascorbic acid-2-phosphate magnesium salt | 1.00 |
| Lauroylhydroxyproline | 1.00 |
| Water soluble collagen (1% aqueous solution) | 2.00 |
| 1,3-butylene glycol | 3.00 |
| Ethanol | 5.00 |
| Purified water | Balance |

While the present invention has been particularly shown and described with reference to specific embodiments thereof, those skilled in the art will appreciate that these specific embodiments are merely preferred embodiments and that the scope of the present invention is not limited thereto. Thus, the scope of the present invention is defined by the appended claims and their equivalents.

## Claims

1. A composition for anti-aging of a skin comprising, as active ingredients, dehydroabietic acid, a stereoisomer thereof, a salt thereof, a hydrate thereof or a solvate thereof; and
compound K represented by following Chemical Formula 2, a stereoisomer thereof, a salt thereof, a hydrate thereof or a solvate thereof

[Chemical Formula 2]

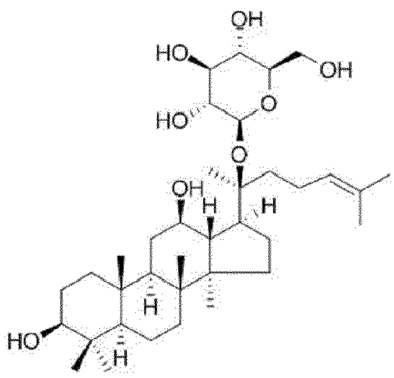

.

2. The composition for anti-aging of the skin according to claim 1,
wherein a weight ratio of dehydroabietic acid, the stereoisomer thereof, the salt thereof, the hydrate thereof or the solvate thereof; and compound K, the stereoisomer thereof, the salt thereof, the hydrate thereof or the solvate thereof in the composition is 1: 1 to 15.

3. The composition for anti-aging of the skin according to claim 2,
wherein the weight ratio is 1: 5 to 10.

4. The composition for anti-aging of the skin according to claim 1,
wherein a content of the active ingredients is 0.0001 to 10 wt% based on the total weight of the composition.

5. The composition for anti-aging of the skin according to claim 1,
wherein the composition is a composition for reducing skin wrinkles.

6. The composition for anti-aging of the skin according to claim 1,
wherein the composition is a composition for enhancing skin elasticity.

**7.** The composition for anti-aging of the skin according to claim 1, wherein the composition prevents skin aging by proliferating fibroblasts.

**8.** The composition for anti-aging of the skin according to claim 1, wherein the composition prevents skin aging by promoting collagen biosynthesis.

**9.** The composition for anti-aging of the skin according to claim 1, wherein the composition prevents skin aging by inhibiting lipofuscin accumulation in the skin.

**10.** The composition for anti-aging of the skin according to any one of claims 1 to 9, wherein the composition is a cosmetic composition.

**11.** The composition for anti-aging of the skin according to any one of claims 1 to 9, wherein the composition is a food composition.

**12.** The composition for anti-aging of the skin according to any one of claims 1 to 9, wherein the composition is a pharmaceutical composition.

**INTERNATIONAL SEARCH REPORT**

International application No.
**PCT/KR2017/009767**

A. CLASSIFICATION OF SUBJECT MATTER
***A61K 8/368(2006.01)i, A61K 8/60(2006.01)i, A23L 33/10(2016.01)i, A61K 31/192(2006.01)i, A61K 31/704(2006.01)i, A61Q 19/00(2006.01)i, A61Q 19/08(2006.01)i***
According to International Patent Classification (IPC) or to both national classification and IPC

B. FIELDS SEARCHED

Minimum documentation searched (classification system followed by classification symbols)
A61K 8/368; A61K 8/63; A61K 8/30; A61K 8/49; A61K 8/24; A61K 47/38; A61Q 17/04; A61K 47/40; A61K 8/36; A61K 8/60; A23L 33/10; A61K 31/192; A61K 31/704; A61Q 19/00; A61Q 19/08

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: skin anti-aging composite, dehydrogenated abietic acid, compound K

C. DOCUMENTS CONSIDERED TO BE RELEVANT

| Category* | Citation of document, with indication, where appropriate, of the relevant passages | Relevant to claim No. |
|---|---|---|
| A | KR 10-2015-0057390 A (AMOREPACIFIC CORPORATION) 28 May 2015<br>See paragraph [0004]; experimental examples 1-3; and claims 1, 9. | 1-12 |
| A | KR 10-2016-0082113 A (AMOREPACIFIC CORPORATION) 08 July 2016<br>See experimental examples 3, 4; claim 1; and table 5. | 1-12 |
| A | US 2010-0183528 A1 (MALONEY, John D. et al.) 22 July 2010<br>See paragraphs [0031], [0032], [0079]-[0081]. | 1-12 |
| A | KR 10-1989-0016959 A (HENKEL AG & CO., KGAA.) 14 December 1989<br>See claims 1, 12. | 1-12 |
| A | KR 10-2013-0106441 A (WACKER CHEMIE AG.) 27 September 2013<br>See claims 1, 3. | 1-12 |

☐ Further documents are listed in the continuation of Box C.   ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11 DECEMBER 2017 (11.12.2017) | **11 DECEMBER 2017 (11.12.2017)** |

| Name and mailing address of the ISA/KR | Authorized officer |
|---|---|
| Korean Intellectual Property Office<br>Government Complex-Daejeon, 189 Seonsa-ro, Daejeon 302-701, Republic of Korea<br>Facsimile No. +82-42-481-8578 | Telephone No. |

Form PCT/ISA/210 (second sheet) (January 2015)

**INTERNATIONAL SEARCH REPORT**
Information on patent family members

International application No.
**PCT/KR2017/009767**

| Patent document cited in search report | Publication date | Patent family member | Publication date |
|---|---|---|---|
| KR 10-2015-0057390 A | 28/05/2015 | NONE | |
| KR 10-2016-0082113 A | 08/07/2016 | NONE | |
| US 2010-0183528 A1 | 22/07/2010 | CA 2747667 A1 | 08/07/2010 |
| | | CA 2747667 C | 22/08/2017 |
| | | EP 2379082 A2 | 26/10/2011 |
| | | EP 2379082 A4 | 11/07/2012 |
| | | EP 2379082 B1 | 10/02/2016 |
| | | ES 2569218 T3 | 09/05/2016 |
| | | US 9125919 B2 | 08/09/2015 |
| | | WO 2010-077971 A2 | 08/07/2010 |
| | | WO 2010-077971 A3 | 04/11/2010 |
| KR 10-1989-0016959 A | 14/12/1989 | CA 1329779 C | 24/05/1994 |
| | | EP 0340592 A2 | 08/11/1989 |
| | | EP 0340592 A3 | 15/05/1991 |
| | | EP 0340592 B1 | 09/03/1994 |
| | | EP 0340592 B2 | 18/03/1998 |
| | | JP 01-319412 A | 25/12/1989 |
| | | JP 2881748 B2 | 12/04/1999 |
| | | US 5002761 A | 26/03/1991 |
| KR 10-2013-0106441 A | 27/09/2013 | CN 103476432 A | 25/12/2013 |
| | | EP 2671596 A1 | 11/12/2013 |
| | | EP 2671596 A4 | 16/07/2014 |
| | | JP 5938587 B2 | 22/06/2016 |
| | | US 2013-0309218 A1 | 21/11/2013 |
| | | WO 2012-105546 A1 | 09/08/2012 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 101349248
- KR 1020140003198
- KR 1020060104161
- KR 100882780
- KR 101401658
- KR 101140039